# EUROPEAN PATENT APPLICATION

(11) **EP 4 516 257 A1**
(43) Date of publication of application: **05.03.2025**
(21) Application number: 23306439.3
(22) Date of filing: 31.08.2023
(51) Int. Cl.: A61B 50/30, A61L 2/26, B65D 37/00, B65D 43/02, B65D 55/08, B65B 55/02, B65D 51/18, B65D 51/20, B65D 75/58

(54) **CONTAINER AND PROTECTIVE COVER FOR CONTAINER FOR TRANSFER OF STERILIZED COMPONENTS**

(71) Applicant: Becton, Dickinson and Company, Franklin Lakes, NJ 07417-1880 (US)
(72) Inventor: Chassin De Kergommeaux, Liliane, 38000 Grenoble (FR); LE LOC'H, Clémentine, 38240 Meylan (FR); EYMERY, Anaïs, 38450 Saint-Georges-de-Commiers (FR)
(74) Representative: Germain Maureau

(57) **Abstract**

A container is configured for transfer of sterilized components from the container into a sterile chamber through a transfer port. The container includes a flexible bag defining an interior cavity, and a connection flange connected to the flexible bag. The connection flange has an opening for accessing the interior cavity of the flexible bag. An access door is connected to the connection flange for sealing the opening. The container includes a protective cover removably connectable to the connection flange and at least partially covering the access door. The protective cover is transparent to permit viewing of the access door.

## Description

### BACKGROUND

### Field of the Disclosure

The present disclosure relates generally to the packaging and transfer of sterile components used in, e.g., medical devices. More particularly, the present disclosure relates to a protective cover for protecting an access lid or door of a container (i.e., a bag) for the transfer of sterilized components (e.g., plunger stoppers).

### Description of the Related Art

As is known in the art, transfer or storage devices for delivery of a medicament, drug, or vaccine (such as, e.g., syringes) utilize a plunger stopper in contact with an inside surface of a generally tubular syringe barrel in order to draw a substance into (or expel a substance from) the device by way of a plunger rod.

Currently, many such devices are filled and assembled using automated filling machines. Not only do such machines improve productivity and accuracy, but they also provide for a substantially sterile and aseptic filling environment. The various components of the devices (e.g., plunger stoppers, syringe barrels, etc.) are separately provided within the filling machines to enable at least some level of automated assembly.

Generally, a plurality of plunger stoppers are initially provided in a substantially flexible bag or similar container to be accessed by the filling machine prior to assembly. The bag generally has an access door that is configured for connecting to a transfer port of an automated filling machine. The bag and its contents are sterilized via, e.g., gamma irradiation, steam, etc. In this way, the plunger stoppers, are able to be directly transferred from a first sterile environment (i.e., the sterilized bag) to a second sterile environment (i.e., the sterile filling machine).

The access door of the transfer bag is generally protected with a protective cover to protect contamination of the access door during shipping and handling prior to connection with the sterile filling machine. Generally, the protective cover is a fabric cover having an elastic band that is configured to fit around a flange of the access door. There are several disadvantages associated with existing protective covers for transfer bags. The fabric material of the protective cover is opaque, thereby preventing visual verification of the integrity of the access door. In order for the user to verify the integrity of the access door, the protective cover must be temporarily removed, thereby exposing the access door to undesired contamination. The elastic band of the protective cover can be installed in multiple positions relative to the access door, leading to low repeatability of preferred installation.

### SUMMARY

In view of the foregoing, there exists a need for an improved protective cover to be utilized in conjunction with a transfer bag in order to protect the access lid or door of the transfer bag during shipping and storage. Desirably, the improved protective cover addresses the shortcomings of existing protective covers to permit visual verification of the integrity of the access door and high repeatability of preferred installation.

In accordance with some non-limiting embodiments or aspects of the present disclosure, provided is a container configured for transfer of sterilized components from the container into a sterile chamber through a transfer port. The container may include a flexible bag defining an interior cavity, and a connection flange connected to the flexible bag. The connection flange may have an opening for accessing the interior cavity of the flexible bag. An access door may be connected to the connection flange for sealing the opening, and a protective cover may be removably connectable to the connection flange to at least partially cover the access door. The protective cover may be transparent.

In accordance with some non-limiting embodiments or aspects of the present disclosure, the connection flange may include at least one radial recess extending radially inward from a perimeter surface of the connection flange. The protective cover may include at least one locking tab configured to be received within the at least one radial recess. The at least one radial recess may be a plurality of radial recesses radially spaced apart about a central axis of the connection flange. The at least one locking tab may be a plurality of locking tabs radially spaced apart about a central axis of the protective cover.

In accordance with some non-limiting embodiments or aspects of the present disclosure, the protective cover may be rotatable relative to the connection flange between an unlocked position, in which the at least one locking tab is radially aligned with the at least one radial recess such that the at least one locking tab is received with the at least one radial recess to permit removal of the protective cover from the connection flange, and a locked position, in which the at least one locking tab is radially offset relative to the at least one radial recess to prevent removal of the protective cover from the connection flange. At least one of the connection flange and the protective cover may have a lock indicator configured to indicate the locked position of the protective cover.

In accordance with some non-limiting embodiments or aspects of the present disclosure, the protective cover may include a viewing window over the at least one locking tab. The viewing window may be configured to permit visual presence of the at least one radial recess on the connection flange when the protective cover is in the unlocked position.

In accordance with some non-limiting embodiments or aspects of the present disclosure, the connection flange may include a perimeter lip. The protective cover may have at least one clip that is configured to snap fit to the perimeter lip of the connection flange. The protective cover may have at least one rib protruding from an inner surface in a direction toward the rigid collar and the access door. The at least one rib may be configured to exert a constant pressure at a connection interface with at least one of the connection flange and the access door.

In accordance with some non-limiting embodiments or aspects of the present disclosure, the protective cover may include a seal configured for engaging at least one of the connection flange and the access door. A pressure control device may be configured for venting excess pressure in a direction from the interior cavity of the flexible bag to atmosphere.

In accordance with some non-limiting embodiments or aspects of the present disclosure, the container may include a tamper evident indicator on the protective cover. The tamper evident indicator may be a tearable shrink sleeve or tab fitted around a perimeter of the protective cover.

In accordance with some non-limiting embodiments or aspects of the present disclosure, the protective cover may be clear or tinted. The protective cover may be made from a rigid plastic material suitable for at least one of a steam sterilization, a gamma sterilization, and ethylene oxide sterilization.

In accordance with some non-limiting embodiments or aspects of the present disclosure, an assembly for transfer of sterilized components from a container into a sterile chamber through a transfer port is provided. The assembly may include a connection flange having an opening, an access door connected to the connection flange for sealing the opening, and a protective cover removably connectable to the connection flange. The protective cover may be transparent.

Various other aspects of the present disclosure are recited in one or more of the following clauses:
Clause 1: A container configured for transfer of sterilized components from the container into a sterile chamber through a transfer port, the container comprising: a flexible bag defining an interior cavity; a connection flange connected to the flexible bag, the connection flange having an opening for accessing the interior cavity of the flexible bag; an access door connected to the connection flange for sealing the opening; and a protective cover removably connectable to the connection flange to at least partially cover the access door, wherein the protective cover is at least partially transparent and/or translucent.
Clause 2: The container according to clause 1, wherein the connection flange comprises at least one radial recess extending radially inward from a perimeter surface of the connection flange, and wherein the protective cover comprises at least one locking tab configured to be received within the at least one radial recess.
Clause 3: The container according to clause 2, wherein the at least one radial recess is a plurality of radial recesses radially spaced apart about a central axis of the connection flange, and wherein the at least one locking tab is a plurality of locking tabs radially spaced apart about a central axis of the protective cover.
Clause 4: The container according to clause 2 or 3, wherein the protective cover is rotatable relative to the connection flange between an unlocked position, in which the at least one locking tab is radially aligned with the at least one radial recess such that the at least one locking tab is received with the at least one radial recess to permit removal of the protective cover from the connection flange, and a locked position, in which the at least one locking tab is radially offset relative to the at least one radial recess to prevent removal of the protective cover from the connection flange.
Clause 5: The container according to any of clauses 2 to 4, wherein the protective cover comprises a viewing window over the at least one locking tab, and wherein the viewing window is configured to permit visual presence of the at least one radial recess on the connection flange when the protective cover is in the unlocked position.
Clause 6: The container according to any of clauses 2 to 5, wherein at least one of the connection flange and the protective cover has a lock indicator configured to indicate the locked position of the protective cover.
Clause 7: The container according to clause 1, wherein the connection flange comprises a perimeter lip, wherein the protective cover comprises at least one clip, and wherein the at least one clip is configured to snap fit to the perimeter lip.
Clause 8: The container according to any of clauses 1 to 7, wherein the protective cover has at least one rib protruding from an inner surface in a direction toward the rigid collar and the access door, wherein the connection flange comprises an annular seal, and wherein the at least one rib is configured to contact the annular seal when the protective cover is connected to the connection flange..
Clause 9: The container according to any of clauses 1 to 8, wherein the protective cover comprises a seal configured for engaging at least one of the connection flange and the access door.
Clause 10: The container according to any of clauses 1 to 9, further comprising a pressure control device configured for venting excess pressure in a direction from the interior cavity of the flexible bag to atmosphere.
Clause 11: The container according to any of clauses 1 to 10, further comprising a tamper evident indicator on the protective cover.
Clause 12: The container according to clause 11, wherein the tamper evident indicator is a tearable shrink sleeve or tab fitted around a perimeter of the protective cover.
Clause 13: The container according to any of clauses 1 to 12, wherein the protective cover is clear.
Clause 14: The container according to any of clauses 1 to 12, wherein the protective cover is tinted.
Clause 15: The container according to any of clauses 1 to 14, wherein the protective cover is made from a rigid plastic material suitable for at least one of a steam sterilization, a gamma sterilization, and ethylene oxide sterilization.
Clause 16: An assembly for transfer of sterilized components from a container into a sterile chamber through a transfer port, the assembly comprising: a connection flange having an opening; an access door connected to the connection flange for sealing the opening; and a protective cover removably connectable to the connection flange, wherein the protective cover is at least partially transparent and/or translucent.
Clause 17: The assembly according to clause 16, wherein the connection flange comprises at least one radial recess extending radially inward from a perimeter surface of the connection flange, and wherein the protective cover comprises at least one locking tab configured to be received within the at least one radial recess.
Clause 18: The assembly according to clause 17, wherein the at least one radial recess is a plurality of radial recesses radially spaced apart about a central axis of the connection flange, and wherein the at least one locking tab is a plurality of locking tabs radially spaced apart about a central axis of the protective cover.
Clause 19: The assembly according to clause 17 or 18, wherein the protective cover is rotatable relative to the connection flange between an unlocked position, in which the at least one locking tab is radially aligned with the at least one radial recess such that the at least one locking tab is received with the at least one radial recess to permit removal of the protective cover from the connection flange, and a locked position, in which the at least one locking tab is radially offset relative to the at least one radial recess to prevent removal of the protective cover from the connection flange.
Clause 20: The assembly according to any of clauses 17 to 19, wherein the protective cover comprises a viewing window over the at least one locking tab, and wherein the viewing window is configured to permit visual presence of the at least one radial recess on the connection flange when the protective cover is in the unlocked position.
Clause 21: The assembly according to any of clauses 17 to 20, wherein at least one of the connection flange and the protective cover has a lock indicator configured to indicate the locked position of the protective cover.
Clause 22: The assembly according to clause 16, wherein the connection flange comprises a perimeter lip, wherein the protective cover comprises at least one clip, and wherein the at least one clip is configured to snap fit to the perimeter lip.
Clause 23: The assembly according to any of clauses 16 to 22, wherein the protective cover has at least one rib protruding from an inner surface in a direction toward the rigid collar and the access door, wherein the connection flange comprises an annular seal, and wherein the at least one rib is configured to contact the annular seal when the protective cover is connected to the connection flange..
Clause 24: The assembly according to any of clauses 16 to 23, wherein the protective cover comprises a first seal configured for engaging at least one of the connection flange and the access door, or wherein the protective cover is configured to engage a second seal on at least one of the connection flange and the access door.
Clause 25: The assembly according to any of clauses 16 to 24, further comprising a pressure control device configured for venting excess pressure in a direction from the opening of the connection flange to atmosphere.
Clause 26: The assembly according to any of clauses 16 to 25, further comprising a tamper evident indicator on the protective cover.
Clause 27: The assembly according to clause 26, wherein the tamper evident indicator is a tearable shrink sleeve or tab fitted around a perimeter of the protective cover.
Clause 28: The assembly according to any of clauses 16 to 27, wherein the protective cover is clear.
Clause 29: The assembly according to any of clauses 16 to 27, wherein the protective cover is tinted.
Clause 30: The assembly according to any of clauses 16 to 29, wherein the protective cover is made from a rigid plastic material suitable for at least one of a steam sterilization, a gamma sterilization, and ethylene oxide sterilization.

Further details and advantages of the present disclosure will be understood from the following detailed description read in conjunction with the accompanying drawings.

### BRIEF DESCRIPTION OF THE DRAWINGS

FIG. 1 is an exploded schematic view of a transfer bag with access door incorporating a protective cover in accordance with an embodiment or aspect of the present disclosure;
FIG. 2 is a perspective view of a protective cover for a transfer bag in accordance with an embodiment or aspect of the present disclosure;
FIG. 3 is a partial side cross-sectional view of the protective cover shown in FIG. 2;
FIG. 4 is a top perspective view of a connection collar of a transfer bag in accordance with an embodiment or aspect of the present disclosure;
FIG. 5 is a bottom view of a connection collar of a transfer bag and a protective cover in accordance with an embodiment or aspect of the present disclosure, with the protective cover shown in an unlocked position;
FIG. 6 is a bottom view of a connection collar of a transfer bag and a protective cover in accordance with an embodiment or aspect of the present disclosure, with the protective cover shown in a locked position;
FIG. 7 is a side view of the connection collar and a side cross-sectional view of the protective cover shown in FIG. 6;
FIG. 8 is an enlarged view of a portion of FIG. 7;
FIG. 9 is a side view of a connection collar of a transfer bag and a protective cover in accordance with an embodiment or aspect of the present disclosure, with the protective cover shown removed from the connection collar;
FIG. 10 is a side view of the connection collar of the transfer bag of FIG. 9, with the protective cover shown connected the connection collar;
FIG. 11 is a partial side cross-sectional view of a protective cover for a transfer bag in accordance with an embodiment or aspect of the present disclosure; and
FIGS. 12A-12C are bottom views of protective covers for a transfer bag in accordance with additional embodiments or aspects of the present disclosure.

### DESCRIPTION OF THE INVENTION

The following description is provided to enable those skilled in the art to make and use the described aspects contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

As used herein, the singular form of "a", "an", and "the" include plural referents unless the context clearly dictates otherwise.

Spatial or directional terms, such as "left", "right", "inner", "outer", "above", "below", and the like, relate to the embodiments or aspects as shown in the drawing figures and are not to be considered as limiting as the embodiments or aspects can assume various alternative orientations.

All numbers used in the specification and claims are to be understood as being modified in all instances by the term "about". By "about" is meant within plus or minus twenty-five percent of the stated value. However, this should not be considered as limiting to any analysis of the values under the doctrine of equivalents.

Unless otherwise indicated, all ranges or ratios disclosed herein are to be understood to encompass the beginning and ending values and any and all subranges or subratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

The terms "first", "second", and the like are not intended to refer to any particular order or chronology, but refer to different conditions, properties, or elements.

All documents referred to herein are "incorporated by reference" in their entirety.

The term "at least" is synonymous with "greater than or equal to".

As used herein, "at least one of' is synonymous with "one or more of'. For example, the phrase "at least one of A, B, or C" means any one of A, B, or C, or any combination of any two or more of A, B, or C. For example, "at least one of A, B, and C" includes A alone; or B alone; or C alone; or A and B; or A and C; or B and C; or all of A, B, and C.

The word "comprising" and "comprises", and the like, does not exclude the presence of elements or steps other than those listed in any claim or the specification as a whole. In the present specification, "comprises" means "includes" and "comprising" means "including".

As used herein, the terms "parallel" or "substantially parallel" mean a relative angle as between two objects (if extended to theoretical intersection), such as elongated objects and including reference lines, that is from 0° to 5°, or from 0° to 3°, or from 0° to 2°, or from 0° to 1°, or from 0° to 0.5°, or from 0° to 0.25°, or from 0° to 0.1°, inclusive of the recited values.

As used herein, the terms "perpendicular", "transverse", "substantially perpendicular", or "substantially transverse" mean a relative angle as between two objects at their real or theoretical intersection is from 85° to 90°, or from 87° to 90°, or from 88° to 90°, or from 89° to 90°, or from 89.5° to 90°, or from 89.75° to 90°, or from 89.9° to 90°, inclusive of the recited values.

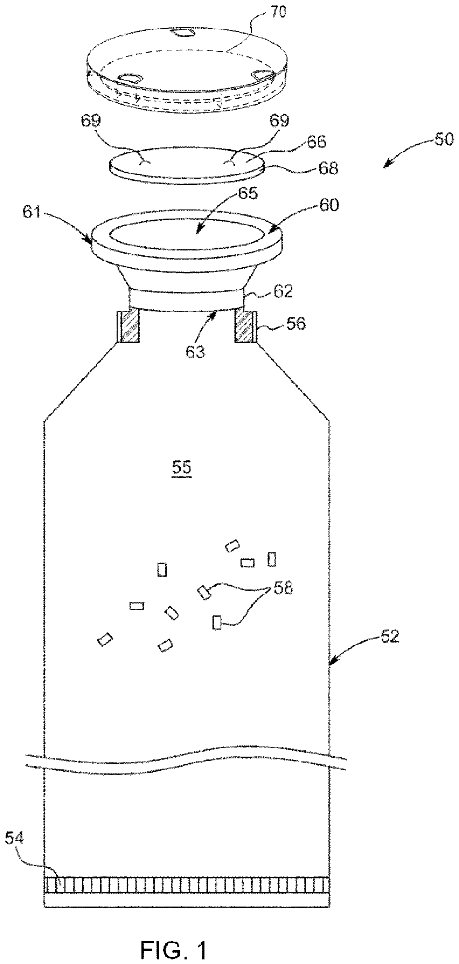

With reference to FIG. 1, a component transfer container 50 in accordance with an embodiment or aspect of the present disclosure is shown. The transfer container 50 is configured for transfer of sterilized components from the transfer container 50 into a sterile chamber of a processing machine through a transfer port. The transfer container 50 includes a flexible primary bag 52 having a distal end 54 and a proximal end 56 and defining an interior cavity 55. The distal end 54 is closed, while the proximal end 56 is open, preferably in the form of an annular opening. In some embodiments or aspects, the flexible primary bag 52 may taper toward the proximal end 56 in the form of, e.g., a bottleneck. The flexible primary bag 52 may be formed of any appropriate flexible material suitable for steam sterilization, gamma sterilization, or ethylene oxide sterilization. Examples of such a flexible material may be, e.g., polyethylene (PE), high-density polyethylene (HDPE), thermoplastic elastomer (TPE), polypropylene, polyvinylidene fluoride (PVDF), etc. Additionally, the interior cavity 55 of the flexible primary bag 52 is sized and configured to hold a plurality of sterilized components 58 therein, such as, e.g., a plurality of plunger stoppers, a plurality of needle covers, a plurality of tip caps, etc.

Referring still to FIG. 1, transfer container 50 further includes a connection flange 60. The connection flange 60 may be formed of any appropriate rigid material suitable for steam sterilization, gamma sterilization, or ethylene oxide sterilization, such as, e.g., polycarbonate (PC), acrylonitrile butadiene styrene (ABS), polyvinylidene fluoride (PVDF), polybutylene terephthalate (PBT), etc. The connection flange 60 is configured to support the locking forces against a door of a transfer portal of a sterile chamber. In some embodiments or aspects, the connection flange 60 includes a distal end 61 configured for a twist-lock connection to the transfer portal. However, it is to be understood that connection flange 60 may be configured for other forms of connection to the transfer portal, such as, e.g., a magnetic connection. The distal end 61 may have an annular seal 67 protruding relative to the distal surface 69 of the distal end 61. The annular seal 67 may be configured for a sealing engagement with a protective cover, as described herein.

As shown in FIG. 1, a proximal end 63 of the connection flange 60 includes an annular neck 62. The proximal end 56 of the flexible bag 52 is affixed to the annular neck 62 by way of a rotational seal. Any appropriate method of sealing such as, e.g., heat sealing, adhesive sealing, etc., may be utilized in forming the rotational seal between the proximal end 56 and the annular neck 62. In this way, the contents of flexible bag 52 (i.e., components 58) may be funneled through an opening 65 formed in the connection flange 60 when the transfer container 50 is coupled to a transfer port.

With continued reference to FIG. 1, the transfer container 50 includes an access door 66. The access door 66 is removably connectable to the connection flange 60 to permit access to the interior cavity 55 of the transfer container 50. In some embodiments or aspects, the access door 66 is configured to seal the transfer container 50 to maintain the sterility of the interior cavity 55 after sterilization. The access door 66 may be completely removable from the connection flange 60 to permit unobstructed access into the interior cavity 55. In some embodiments or aspects, the access door 66 may remain connected to the connection flange 60 and may be movable between an open position to permit access into the interior cavity 55, and a closed position to seal the connection flange 60 and prevent access to the interior cavity 55. The access door 66 has a sealing surface 68 configured for a sealed engagement with the connection flange 60. The sealing surface 68 may be an O-ring or a similar sealing structure configured for maintaining the sterility of the interior cavity 55 by preventing ingress of outside contaminants into the interior cavity 55.

The access door 66 may include one or more handling features 69 to facilitate connecting or disconnecting the access door 66 to and from the connection flange 60. For example, the one or more handling features 69 may be one or more handles, pull tabs, or other structures. The access door 66 may be formed of any appropriate rigid material suitable for steam sterilization, gamma sterilization, or ethylene oxide sterilization, such as, e.g., polycarbonate (PC), acrylonitrile butadiene styrene (ABS), polyvinylidene fluoride (PVDF), polybutylene terephthalate (PBT), etc.

With continued reference to FIG. 1, the transfer container 50 includes a protective cover 70 removably connectable to the connection flange 60. The protective cover 70 is configured for extending over the access door 66 to protect the access door 66 during shipping and handling. The protective cover 70 is shaped to correspond to an outer shape of the connection flange 60. The protective cover 70, along with the connection flange 60 and the access door 66, defines an assembly for transfer of sterilized components 58 from the transfer container 50 into a sterile chamber.

As discussed herein, at least a portion of the protective cover 70 is desirably made from a transparent and/or translucent material in order to permit visual verification of the condition of the access door 66. For example, at least a portion of the protective cover 70 may be formed of any appropriate rigid material transparent and/or translucent suitable for steam sterilization, gamma sterilization, or ethylene oxide sterilization. In some embodiments or aspects, the protective cover 70 may be formed from a clear transparent or a clear translucent material. In other embodiments or aspects, the protective cover 70 may be formed from a tinted transparent material or a tinted translucent material in order to provide a visual contrast relative to the access door 66. In this manner, presence of the protective cover 70 can be easily verified by visually observing the tinted color of the protective cover 70.

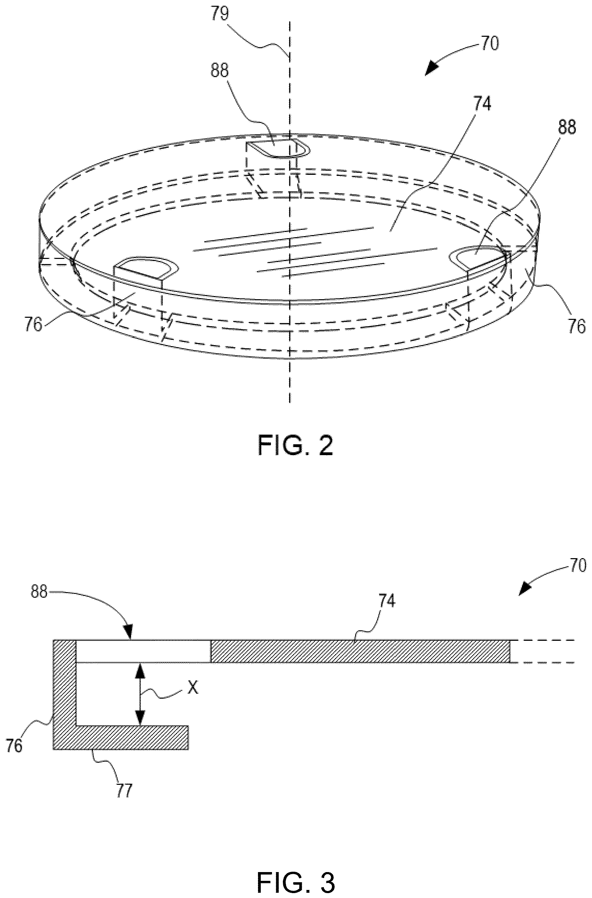

With reference to FIG. 2, the protective cover 70 is shown in accordance with one embodiment or aspect of the present disclosure. The protective cover 70 has a cover face 74 that is configured to cover the connection flange 60 and the access door 66. In some embodiments or aspects, the cover face 74 is shaped to correspond to an outer shape of the connection flange 60. While the cover face 74 may be substantially planar, other non-planar shapes are also possible. A portion of an inner surface of the cover face 74 is configured to contact the annular seal 67 of the connection flange 60 when the protective cover 70 is connected to the connection flange 60. In this manner, a sealed connection can be made between the protective cover 70 and the connection flange 60.

With continued reference to FIG. 2, the protective cover 70 has an annular rim 76 protruding from the cover face 74. In some embodiments or aspects, the annular rim 76 has a first portion that protrudes away from the cover face 74 in a direction that is substantially perpendicular to a plane defined by the cover face 74 and a second portion that protrudes in a direction toward a central axis 79 of the protective cover 70.

With continued reference to FIG. 2 and with reference to FIG. 3, the annular rim 76 may have an "L" shape, although other shapes of the annular rim 76 are not precluded. The annular rim 76 may have a first end 76a connected to the cover face 74 and a second free end 76b. The second free 76b end may have at least one locking tab 77 that protrudes toward the central axis 79 and is spaced apart from the cover face 74 by a distance X (shown in FIG. 3). The at least one locking tab 77 may be a plurality of locking tabs 77 spaced apart from each other around a perimeter of the cover face 74 about the central axis 79. In some embodiments or aspects, the locking tabs 77 may have equal angular spacing from each other around a perimeter of the cover face 74. In other embodiments or aspects, the locking tabs 77 may have unequal angular spacing therebetween.

The annular rim 76 may be configured to interact with at least a portion of the connection flange 60 for removably securing the protective cover 70 to the connection flange 60. For example, the annular rim 76, including the at least one locking tab 77, may be configured to interact with corresponding locking features on the connection flange 60 to removably secure the protective cover 70 to the connection flange 60.

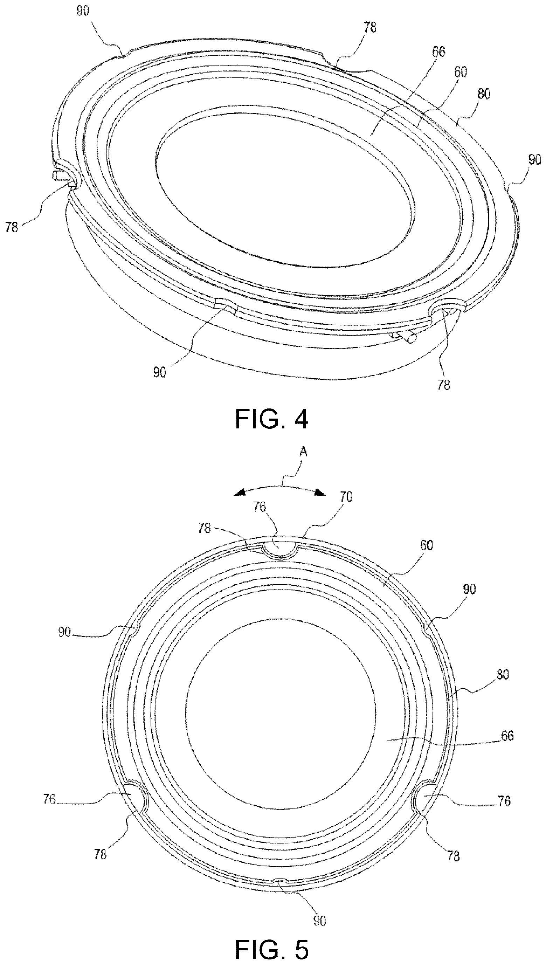

With reference to FIG. 4, the connection flange 60 may have at least one radial recess 78 extending radially inward from a perimeter surface 80 of the connection flange 60. Each radial recess 78 may be shaped to correspond to a shape of the locking tab 77 on the protective cover 70. Each radial recess 78 is configured to receive the corresponding locking tab 77 when the protective cover 70 is aligned for connection with the connection flange 60. The number and spacing of the radial recesses 78 on the connection flange 60 corresponds to the number and spacing of the locking tabs 77 on the protective cover 70. The annular seal 67 is positioned radially inward of the perimeter surface 80 and protrudes distally thereto.

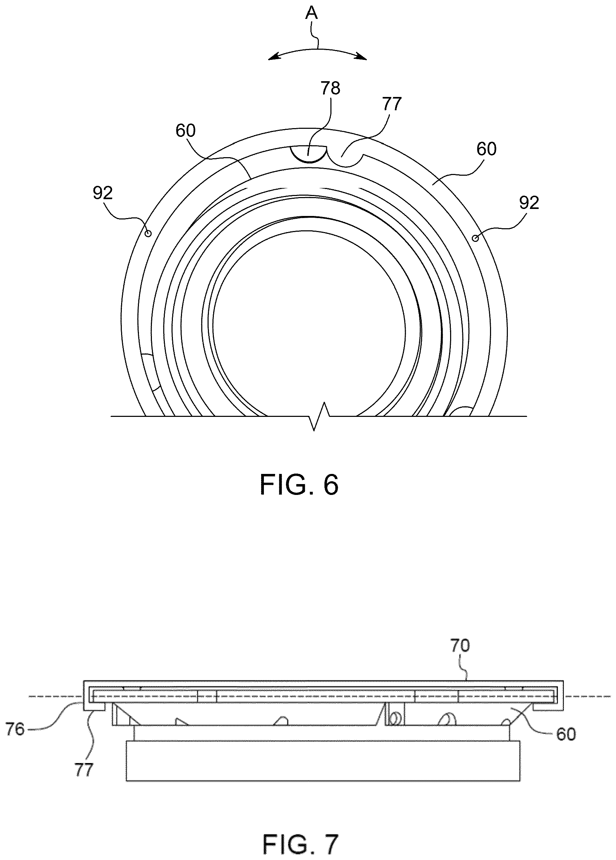

In some embodiments or aspects, the protective cover 70 is rotatable relative to the connection flange 60 about the central axis 79 between an unlocked position (FIG. 5), in which each of locking tabs 77 is radially aligned with the radial recesses 78 such that the locking tab 77 is received with the at least one radial recess 78 to permit removal of the protective cover 70 from the connection flange 60, and a locked position (FIG. 6), in which each of the locking tabs 77 is radially offset relative to the radial recesses 78 to prevent removal of the protective cover 70 from the connection flange 60. For example, the protective cover 70 may be rotatable about the central axis 79 in a direction of arrow A (shown in FIG. 5). In some embodiments or aspects, the protective cover 70 may be rotatable in a first (e.g., clockwise) direction or a second (e.g., counterclockwise) direction to either lock the protective cover 70 from the unlocked position to the locked position, or to unlock the protective cover 70 from the locked position to the unlocked position. In other embodiments or aspects, the protective cover 70 may be rotatable in a single direction to lock the protective cover 70 and an opposite direction to unlock the protective cover 70. For example, the protective cover 70 may be rotatable in a first direction to lock the protective cover 70 from the unlocked position to the locked position, and in a second direction opposite the first direction to unlock the protective cover 70 from the locked position to the unlocked position.

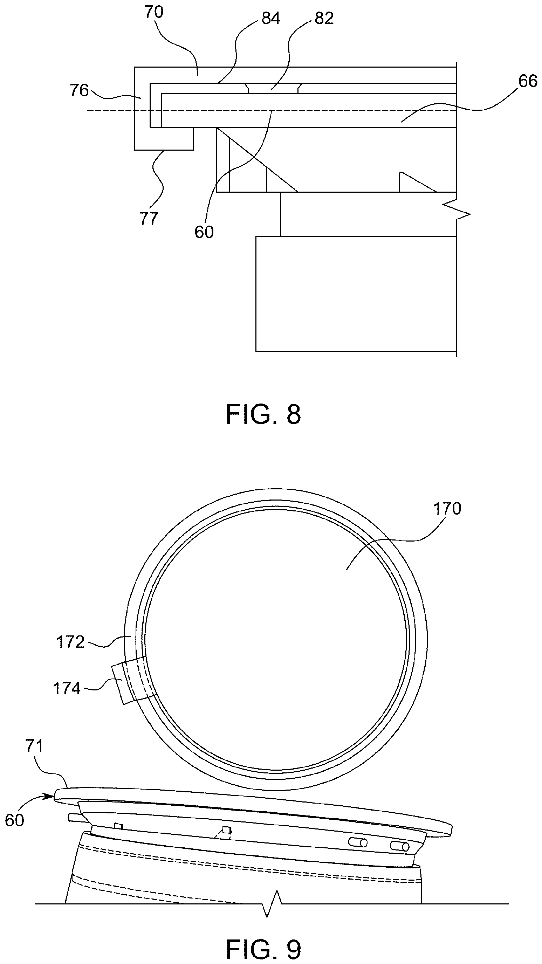

With reference to FIGS. 7-8, rotation of the protective cover 70 to the locked position may position the locking tab 77 of each protrusion 76 under the connection flange 60 to prevent removal of the protective cover 70 from the connection flange 60. Distance X between the inner surface of the cover face 74 and the locking tab 77 (shown in FIG. 3) may correspond to a thickness of the connection flange 70. In some embodiments or aspects, and as shown in FIG. 8), the protective cover 70 has at least one rib 82 protruding from an inner surface 84 of the cover face 74 in a direction toward the connection flange 60 and the access door 66. The at least one rib 82 may be an annular structure that is aligned with the annular seal 67 on the connection flange 60. When the protective cover 70 is in the locked position, the at least one rib 82 is configured to exert a constant pressure on the annular seal 67 to seal the interface between the protective cover 70 and the connection flange 60.

With reference to FIG. 2, the protective cover 70 may optionally include at least one viewing window 88 over the at least one protrusion 76. The viewing window 88 may extend through the cover face 74 such that the at least a portion of the protrusion 76 may be visible through the viewing window 88. The viewing window 88 may be configured to permit confirmation of visual presence of the at least one radial recess 78 on the connection flange 70 when the protective cover 70 is in the unlocked position. In this manner, the viewing window helps with proper alignment of the protective cover 70 relative to the connection flange 60 during initial connection of the protective cover 70 to the connection flange 60.

At least one of the connection flange 60 and the protective cover 70 may have a lock indicator configured to indicate the locked position of the protective cover 70. For example, and with reference to FIGS. 5-6, the connection flange 60 may have a first lock indicator 90 and the protective cover 70 may have a second lock indicator 92. In some embodiments or aspects, the first and second lock indicators 90, 92 may be dots, recesses, protrusions, or other structures on the connection flange 60 and the protective cover 70 that provide a visual indication of a rotational alignment between the connection flange 60 and the protective cover 70. For example, the first lock indicator 90 and the second lock indicator 92 may be rotationally aligned only when the protective cover 70 is in the locked position with the connection flange 60.

With reference to FIGS. 9-10, a protective cover 170 is shown in accordance with another embodiment or aspect of the present disclosure. The connection flange 60 includes a perimeter lip 71, and the protective cover 170 has at least one clip 172 that is configured for removably connecting with the perimeter lip 71 of the connection flange 70. The at least one clip 172 may be a continuous clip that extends around an entire perimeter of the protective cover 170 to permit the protective cover 170 to snap over the perimeter lip 71. In some embodiments or aspects, the at least one clip 172 may be a plurality of clips that are radially spaced from each other around a perimeter of the protective cover 170. A tab 174 may be provided on an outer portion of the protective cover 170 to facilitate with removal of the protective cover 170 from the connection flange 60.

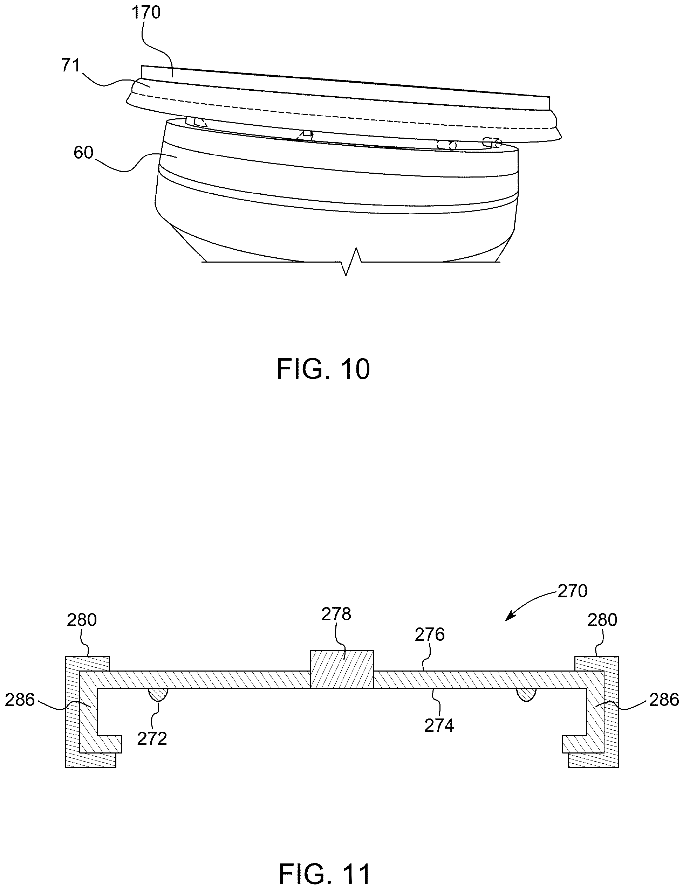

With reference to FIG. 11, a protective cover 270 is shown in accordance with another embodiment or aspect of the present disclosure. The protective cover 270 has a seal 272 configured for engaging at least one of the connection flange 70 and the access door 66 (shown in FIG. 1). The seal 272 may be provided on an inner surface 274 of the cover face 276. In some embodiments or aspects, the seal 272 may be an elastomeric O-ring.

With continued reference to FIG. 11, the protective cover 270 may have a pressure control device 278 configured for venting excess pressure in a direction from the interior cavity of the transfer bag 50 (shown in FIG. 1) to atmosphere. The pressure control device 278 may be a one-way valve.

With continued reference to FIG. 11, the protective cover 270 may have a tamper evident indicator 280. The tamper evident indicator 280 may be a tearable shrink sleeve or tab fitted around a perimeter of the protective cover 270, such as on at least one of the protrusions 286. The tamper evident indicator 280 may prevent removal of the protective cover 270 from the connection flange 70 prior to removal of the tamper evident indicator 280.

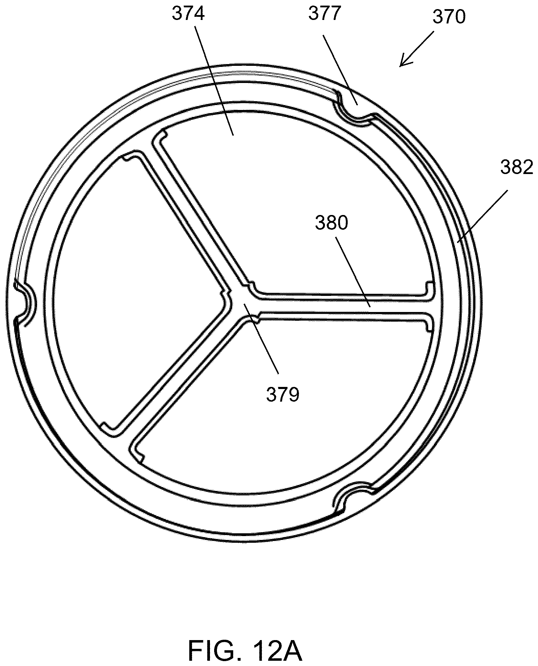

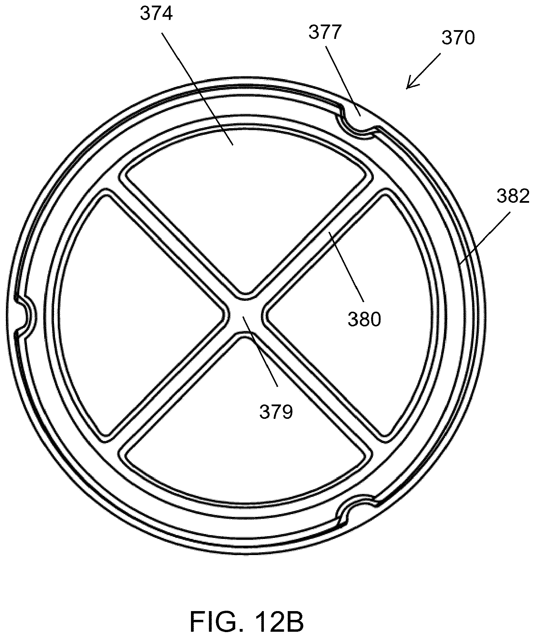

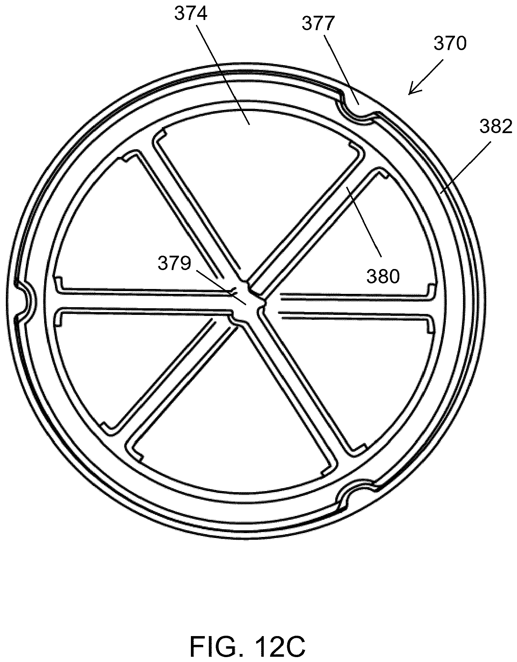

With reference to FIGS. 12A-12C, a protective cover 370 is shown in accordance with additional embodiments or aspects of the present disclosure. The protective covers 370 shown in FIGS. 12A-12C may have the same features discussed herein with reference to the embodiments or aspects shown in FIGS. 2-11. In addition to the features of any of these embodiments or aspects, the protective covers 370 shown in FIGS. 12A-12C further include at least one reinforcement rib 380 extending across at least a portion of the cover face 374 and configured to increase the structural rigidity of the protective cover 370. In some embodiments or aspects, the protective cover 370 may have 3, 4, 6, or any other number of reinforcement ribs 380 protruding radially inwardly from an outer rim 382 of the protective cover 370. The reinforcement ribs 380 may be spaced apart from each other around a perimeter of the cover face 374 about the central axis 379. In some embodiments or aspects, the reinforcement ribs 380 may have equal angular spacing from each other around a perimeter of the cover face 374. In other embodiments or aspects, the reinforcement ribs 380 may have unequal angular spacing therebetween. The reinforcement ribs 380 may be aligned with the locking tabs 377, such as by being positioned between the locking tabs 377 or in line with the locking tabs 377.

While several embodiments of transfer containers and protective cover arrangements are shown in the accompanying figures and described hereinabove in detail, other embodiments will be apparent to, and readily made by, those skilled in the art without departing from the scope of the invention. For example, it is to be understood that this disclosure contemplates, to the extent possible, that one or more features of any embodiment can be combined with one or more features of any other embodiment. Accordingly, the foregoing description is intended to be illustrative rather than restrictive.

## Claims

1. A container configured for transfer of sterilized components from the container into a sterile chamber through a transfer port, the container comprising:
a flexible bag defining an interior cavity;
a connection flange connected to the flexible bag, the connection flange having an opening for accessing the interior cavity of the flexible bag;
an access door connected to the connection flange for sealing the opening; and
a protective cover removably connectable to the connection flange to at least partially cover the access door,
wherein the protective cover is at least partially transparent and/or translucent.

2. The container according to claim 1, wherein the connection flange comprises at least one radial recess extending radially inward from a perimeter surface of the connection flange, and wherein the protective cover comprises at least one locking tab configured to be received within the at least one radial recess.

3. The container according to claim 2, wherein the at least one radial recess is a plurality of radial recesses radially spaced apart about a central axis of the connection flange, and wherein the at least one locking tab is a plurality of locking tabs radially spaced apart about a central axis of the protective cover.

4. The container according to claim 2 or 3, wherein the protective cover is rotatable relative to the connection flange between an unlocked position, in which the at least one locking tab is radially aligned with the at least one radial recess such that the at least one locking tab is received with the at least one radial recess to permit removal of the protective cover from the connection flange, and a locked position, in which the at least one locking tab is radially offset relative to the at least one radial recess to prevent removal of the protective cover from the connection flange.

5. The container according to any of claims 2 to 4, wherein the protective cover comprises a viewing window over the at least one locking tab, and wherein the viewing window is configured to permit visual presence of the at least one radial recess on the connection flange when the protective cover is in the unlocked position.

6. The container according to any of claims 2 to 5, wherein at least one of the connection flange and the protective cover has a lock indicator configured to indicate the locked position of the protective cover.

7. The container according to claim 1, wherein the connection flange comprises a perimeter lip, wherein the protective cover comprises at least one clip, and wherein the at least one clip is configured to snap fit to the perimeter lip.

8. The container according to any of claims 1 to 7, wherein the protective cover has at least one rib protruding from an inner surface in a direction toward the rigid collar and the access door, wherein the connection flange comprises an annular seal, and wherein the at least one rib is configured to contact the annular seal when the protective cover is connected to the connection flange.

9. The container according to any of claims 1 to 8, wherein the protective cover comprises a seal configured for engaging at least one of the connection flange and the access door.

10. The container according to any of claims 1 to 9, further comprising a pressure control device configured for venting excess pressure in a direction from the interior cavity of the flexible bag to atmosphere.

11. The container according to any of claims 1 to 10, further comprising a tamper evident indicator on the protective cover.

12. The container according to claim 11, wherein the tamper evident indicator is a tearable shrink sleeve or tab fitted around a perimeter of the protective cover.

13. The container according to any of claims 1 to 12, wherein the protective cover is clear.

14. The container according to any of claims 1 to 12, wherein the protective cover is tinted.

15. The container according to any of claims 1 to 14, wherein the protective cover is made from a rigid plastic material suitable for at least one of a steam sterilization, a gamma sterilization, and ethylene oxide sterilization.

16. An assembly for transfer of sterilized components from a container into a sterile chamber through a transfer port, the assembly comprising:
a connection flange having an opening;
an access door connected to the connection flange for sealing the opening; and
a protective cover removably connectable to the connection flange to at least partially cover the access door,
wherein the protective cover is at least partially transparent and/or translucent.

17. The assembly according to claim 16, wherein the connection flange comprises at least one radial recess extending radially inward from a perimeter surface of the connection flange, and wherein the protective cover comprises at least one locking tab configured to be received within the at least one radial recess.

18. The assembly according to claim 17, wherein the at least one radial recess is a plurality of radial recesses radially spaced apart about a central axis of the connection flange, and wherein the at least one locking tab is a plurality of locking tabs radially spaced apart about a central axis of the protective cover.

19. The assembly according to claim 17 or 18, wherein the protective cover is rotatable relative to the connection flange between an unlocked position, in which the at least one locking tab is radially aligned with the at least one radial recess such that the at least one locking tab is received with the at least one radial recess to permit removal of the protective cover from the connection flange, and a locked position, in which the at least one locking tab is radially offset relative to the at least one radial recess to prevent removal of the protective cover from the connection flange.

20. The assembly according to any of claims 17 to 19, wherein the protective cover comprises a viewing window over the at least one locking tab, and wherein the viewing window is configured to contact the annular seal when the protective cover is connected to the connection flange.

21. The assembly according to any of claims 17 to 20, wherein at least one of the connection flange and the protective cover has a lock indicator configured to indicate the locked position of the protective cover.

22. The assembly according to claim 16, wherein the connection flange comprises a perimeter lip, wherein the protective cover comprises at least one clip, and wherein the at least one clip is configured to snap fit to the perimeter lip.

23. The assembly according to any of claims 16 to 22, wherein the protective cover has at least one rib protruding from an inner surface in a direction toward the rigid collar and the access door, wherein the connection flange comprises an annular seal, and wherein the at least one rib is configured to exert a constant pressure at a connection interface with at least one of the connection flange and the access door.

24. The assembly according to any of claims 16 to 23, wherein the protective cover comprises a seal configured for engaging at least one of the connection flange and the access door.

25. The assembly according to any of claims 16 to 24, further comprising a pressure control device configured for venting excess pressure in a direction from the opening of the connection flange to atmosphere.

26. The assembly according to any of claims 16 to 25, further comprising a tamper evident indicator on the protective cover.

27. The assembly according to claim 26, wherein the tamper evident indicator is a tearable shrink sleeve or tab fitted around a perimeter of the protective cover.

28. The assembly according to any of claims 16 to 27, wherein the protective cover is clear.

29. The assembly according to any of claims 16 to 27, wherein the protective cover is tinted.

30. The assembly according to any of claims 16 to 29, wherein the protective cover is made from a rigid plastic material suitable for at least one of a steam sterilization, a gamma sterilization, and ethylene oxide sterilization.
